# EUROPÄISCHE PATENTSCHRIFT

(11) **EP 1 509 200 B1**
(45) Veröffentlichungstag und Bekanntmachung des Hinweises auf die Patenterteilung: **03.09.2008**
(21) Anmeldenummer: 03727449.5
(22) Anmeldetag: 08.05.2003
(51) Int. Cl.: A61K 9/00

(54) **FILMFÖRMIGE, ZERFALLSFÄHIGE ZUBEREITUNGEN ZUR WIRKSTOFF-FREISETZUNG UND VERFAHREN ZU DEREN HERSTELLUNG**
FILM-SHAPED, DISSOLVABLE PREPARATIONS FOR ACTIVE SUBSTANCE RELEASE AND METHOD FOR THE PRODUCTION THEREOF
PREPARATIONS POUVANT ETRE DISSOUTES SOUS FORME DE FILM POUR LA LIBERATION DE SUBSTANCES ACTIVES ET PROCEDE DE PRODUCTION DESDITES PREPARATIONS

(30) Priorität: 04.06.2002 DE 10224607
(43) Veröffentlichungstag der Anmeldung: 02.03.2005
(73) Patentinhaber: LTS LOHMANN Therapie-Systeme AG, 56626 Andernach (DE)
(72) Erfinder: VON FALKENHAUSEN, Christian, 53340 Meckenheim (DE); SEIBERTZ, Frank, 56598 Rheinbrohl (DE); KRUMME, Markus, Randolph, NJ 07869 (US)
(74) Vertreter: Flaccus, Rolf-Dieter
(86) Internationale Anmeldenummer: PCT/EP2003/004806
(87) Internationale Veröffentlichungsnummer: WO 2003/101420

(56) Entgegenhaltungen:
- WO-A-02/02085
- WO-A-03/070227
- DE-A- 19 837 073
- US-A- 6 072 100
- US-A- 6 153 222
- US-A1- 2001 006 677

## Beschreibung

Die Erfindung betrifft filmförmige, in wässrigen Medien zerfallsfähige Zubereitungen, die auf der Basis von wasserlöslichen Polymeren hergestellt sind und zur Verabreichung von Stoffen an den menschlichen oder tierischen Körper verwendet werden können. Die Erfindung betrifft ferner Verfahren für die Herstellung solcher Zubereitungen, sowie deren Verwendung als pharmazeutische Darreichungsformen.

Flächenförmige, in wässriger Umgebung zerfallende Darreichungsformen, insbesondere orale Darreichungsformen, mittels derer eine rasche Freisetzung von Wirkstoffen in der Mundhöhle oder in anderen Körperöffnungen bzw. Körperhöh-lungen ermöglicht wird, sind bereits bekannt . Diese Darreichungsformen können beispielsweise in Form von Oblaten ("wafer") gestaltet sein. Aufgrund ihrer geringen Schichtdicke und Zerfallsfähigkeit oder Auflösbarkeit eigenen sich diese filmförmigen, flachen Darreichungsformen insbesondere zur raschen Freisetzung von Medikamenten und anderen Wirkstoffen im Mundraum.

Flächenförmige, oblatenartige Filme zur Abgabe von Substanzen an den menschlichen oder tierischen Organismus sind in der Regel aus filmbildende, wasserlöslichen Polymeren aufgebaut. Kommen diese Wafer mit Wasser oder einer Körperflüssigkeit (z. B. Speichel) in Kontakt, lösen sich die Polymere und die Arzneiform zerfällt unter Freisetzung des Wirkstoffes. Dabei zerfällt der Wafer umso schneller, je schneller die wässrige Flüssigkeit auch zu den inneren Bereichen des Wafers vordringt. Deshalb nimmt die Zerfallsge-schwindigkeit mit zunehmender Schichtdicke ab. Die Dicke solcher System ist nun stark durch die Menge und Art des zu führenden Wirkstoff bedingt. Dicke Wafer haben (siehe z.B. US2001/0006677, US6,072,100 und US 6,153,222) hierbei die unangenehme Eigenschaft, aufgrund ihrer flächigen Form und der Verzögerung des Zerfalls am Gaumen festzukleben. Dies ist einerseits durch die sich oberflächlich lösenden Polymerschichten bedingt, welche einen klebrigmatschigen Film bilden, andererseits haften die Darreichungsformen bei Kontakt mit der Mundschleimhaut wegen des Druckgefälles von Ober- zu Unterseite am Gaumen fest.

Durch die Eigenschaft dieser filmförmigen Arzneiformen, am Gaumen und anderen Oberflächen der Mundschleimhaut festzukleben, kann bei der betreffenden Person bzw. beim Patienten eine unangenehme Empfindung hervorrufen werden, d.h. das durch diese "Wafer" erzeugte "mouthfeel" ist unangenehm oder störend und deshalb verbesserungsbedürftig.

Es war deshalb die Aufgabe der vorliegenden Erfindung, eine filmförmige Zubereitung der vorstehend genannten Art bereitzustellen, welche die bekannten Vorteile schnell zerfallender Darreichungsformen aufweist, sich zugleich aber durch verbesserte Zerfallseigenschaften auszeichnet, so daß eine beschleunigte Wirkstofffreisetzung resultiert. Außerdem bestand die Aufgabe darin, die Tendenz zum Anhaften oder Festhaften an die Mundschleimhaut zu verringern, so daß im Falle der Verwendung als orale Darreichungsform ein verbessertes "Mouthfeel" resultiert.
Eine weitere Aufgabe bestand darin, Verfahren anzugeben, mittels derer filmförmige Zubereitungen mit den genannten verbesserten Eigenschaften erhalten werden können.

Überraschenderweise wird die Aufgabe dadurch gelöst, daß eine filmförmige Zubereitung mit den Merkmalen gemäß Oberbegriff des Hauptanspruchs zusätzlich eine oder mehrere Komponente enthält, welche bei Einwirkung von Feuchtigkeit oder in Gegenwart eines wässrigen Mediums oder bei Temperaturänderung ein Gas erzeugen. Dieses Gas wird aus der Zubereitung freigesetzt, beispielsweise wenn sie in die Mundhöhle appliziert wird und mit Speichelflüssigkeit in Kontakt kommt. Die Bildung von Gasbläschen während der oralen Einnahme einer filmförmigen Zubereitung ("Wafer") bewirkt, daß die innere Struktur des Wafers stark beeinträchtigt wird und er schnell in mehrere Fragmente zerfällt. Der Wafer wird also durch die in seinem Inneren entstehenden Gasbläschen geradezu gesprengt. Hierdurch vergrößert sich schlagartig die zur Verfügung stehende Oberfläche, was zu einer beschleunigten Wirkstofffreisetzung führt.
Darüber hinaus wird durch das Entweichen von Gasblasen an den Oberflächen des Wafers verhindert, dass der Wafer auf der Schleimhaut anhaftet. Dies wiederum unterstützt die Zufuhr weiterer Flüssigkeit an beider Seiten des Wafers. Im Falle einer Anhaftung, wie dies bei herkömmlichen Wafern auftreten kann, würde dagegen nur eine Seite für eine vermehrte Flüssigkeitsaufnahme zur Verfügung stehen.

Im Fall einer oralen Applikation der erfindungsgemäßen Wafer bewirkt das Entweichen von Gasblasen an den Oberflächen des Wafers außerdem ein verbessertes "mouthfeel", da der Wafer nicht an der Mundschleimhaut oder Zunge anhaften kann, sondern durch das Zerplatzen der Blasen ständig von der Schleimhaut abgelöst wird.

Die Aufgabe wird des weiteren gelöst durch die in den Ansprüchen 1 bis 5 beschriebenen Herstellungsverfahren.
Diese ermöglichen die Herstellung von in wässrigen Medien zerfallsfähigen, filmförmigen Zubereitungen gemäß Hauptanspruch. Dabei besteht ein wesentlicher Vorteil darin, daß bei der Herstellung der Zubereitungen gasbildende, durch Wasser oder Feuchtigkeit aktivierbare Komponenten hinzugefügt werden können, ohne daß es während der Herstellung zu einer vorzeitigen Gasentwicklung kommt.

Die erfindungsgemäßen Zubereitungen sind flächenförmige Filme, die mindestens ein wasserlösliches Polymer enthalten. Das wasserlösliche Polymer bzw. die Polymere bildet/ bilden die Grundstruktur, auch Matrix genannt, der Zubereitungen. Die Zusammensetzung der Matrix kann ferner Hilfsstoffe verschiedenster Art umfassen, mittels welcher die chemischen und physikalischen Eigenschaften der Filme zusätzlich beeinflußt werden können. Der Gewichtsanteil des/ der wasserlöslichen Polymers/Polymere liegt vorzugsweise im Bereich von 10 bis 95 Gew.-%, besonders bevorzugt im Bereich von 15 bis 70 Gew.-%, jeweils bezogen auf die gesamte Zubereitung (Trockenmasse).

Die verwendeten matrixbildenden polymeren Bestandteile sind hierbei wasserlöslich oder zumindest teilweise wasserlöslich; sie können thermoplastisch oder nicht thermoplastisch sein. Thermoplastische Formulierungen können bei der Herstellung der filmförmigen Zubereitungen unter Hitze extrudiert werden, während nicht-thermoplastische Polymere als hochviskose Lösung extrudiert werden können.
Unter teilweiser Wasserlöslichkeit wird die Eigenschaft vieler Polymere verstanden, in Wasser oder wässrigen Medien quellbar zu sein. Dabei dringen die Wassermoleküle langsam in das Polymermaterial ein, wobei unter Quellung ein Gel entsteht. Ein anschließender Zerfall des gequollenen Gels zu einer echten Lösung tritt nicht auf. Dies ist insbesondere bei Polymeren mit sehr hoher Molekülmasse oder bei vernetzten Polymeren der Fall.

Folgende Polymere können dabei Verwendung finden: Polyvinylalkohol (PVA), Polyethylenoxide, Copolymer aus Methylvinylether und Maleinsäure, Cellulosederivate wie Hydroxypropylmethylcellulose (HPMC), Hydroxypropylcellulose (HPC), Natrium-Carboxymethylcellulose (NaCMC), Methylcellulose (MC), Hydroxyethylcellulose (HEC), Hydroxypropylethylcellulose (HPEC), Stärke und deren Derivate, Gelatinen, Polyvinylpyrrolidone (PVP), Gummi arabicum, Pullulan oder Acrylate. Auch Mischungen aus zwei oder mehreren der genannten Polymere können verwendet werden.

Als Hilfsstoffe, welche dem Fachmann grundsätzlich bekannt sind, können vorteilhaft Stoffe aus folgenden Gruppen Verwendung finden: Füllstoffe wie z.B. SiO₂; Farbstoffe wie Chinolingelb oder TiO₂; Sprengmittel bzw. Dochtmittel, die Wasser in die Matrix hineinziehen und die Matrix von innen her sprengen, wie z. B. Aerosil; Emulgatoren wie Tween (polyethoxylierte Sorbitanfettsäureester), Brij (polyethoxylierte Fettalkohole); Süßstoffe wie Aspartam, Natriumcyclamat und Sacharin; Weichmacher wie PEG (Polyethylenglykol) oder Glycerin; Konservierungsmittel wie beispielsweise Sorbinsäure oder deren Salze. Der Anteil dieser Hilfsstoffe kann vorzugsweise bis zu 30 Gew.-% betragen, insbesondere 1 bis 20 Gew.-%, jeweils bezogen auf die gesamte Zubereitung.

Komponenten, welche bei Einwirkung von Feuchtigkeit oder in Gegenwart eines wässrigen Mediums oder bei Temperaturänderung ein Gas erzeugen, sind dem Fachmann grundsätzlich bekannt. Vorzugsweise enthalten die erfindungsgemäßen filmförmigen Zubereitungen eine oder mehrere gasbildende Komponente(n), die aus der Gruppe ausgewählt ist/sind, die Carbonate, insbesondere Natriumcarbonat, Ammoniumcarbonat, Magnesiumcarbonat, Kaliumcarbonat, und Hydrogencarbonate, insbesondere Natriumhydrogencarbonat, und Säuren, insbesondere Carbonsäuren wie Citronensäure, Äpfelsäure, Essigsäure, Milchsäure, Fumarsäure, Gluconsäure, Weinsäure, sowie Säureregulatoren, insbesondere Salze der Essigsäure, Natriumdihydrogen- oder Dinatriumhydrogenphosphat, Natriumtartrat, Natriumascorbat, umfaßt.

Vorzugsweise wird die Gasbildung durch eine Kombination zweier oder mehrerer Komponenten bewirkt, insbesondere durch die Kombination eines Erdalkali- oder Alkalimetallcarbonates oder Alkalimetallhydrogencarbonates mit einer Carbonsäure. Als Carbonate oder Hydrogencarbonate kommen insbesondere Natriumhydrogencarbonat, Natriumcarbonat, Kaliumcarbonat und Kaliumhydrogencarbonat in Betracht. Unter den Carbonsäuren werden insbesondere diejenigen aus der Citronensäure, Äpfelsäure, Essigsäure, Milchsäure, Fumarsäure, Gluconsäure und Weinsäure umfassenden Gruppe bevorzugt. Besonders bevorzugt ist eine Kombination von Natriumhydrogencarbonat mit Citronensäure.

Der Anteil der gasbildenden Komponente(n) kann bis zu 70 Gew.-% betragen, bezogen auf die Trockenmasse der Zubereitung; vorzugsweise liegt der Anteil im Bereich von 5 bis 60 Gew.-%.

Im Falle der oben genannten gasbildenden Komponenten ist das unter Einwirkung eines wässrigen Mediums entstehende Gas Kohlendioxid. Dies schließt aber nicht aus, daß bei Verwendung einer anderen gasbildenden Verbindung bzw. eines Gemisches von Verbindungen ein andersartiges Gas entsteht, wie z. B. Stickstoff. Sofern CO₂ das in Gegenwart von Wasser oder eines wässrigen Mediums gebildete Gas darstellt, ergibt sich als ein weiterer Vorteil der erfindungsgemäßen filmförmigen Darreichungsformen eine verbesserte Wirkstoffaufnahme in den Körper. Gemäß einer bevorzugten Ausführungsform zeichnen sich die erfindungsgemäßen Zubereitungen dadurch aus, daß sie in Gegenwart von Wasser oder eines wässrigen Mediums ein saures Milieu bilden.

Neben den genannten an der Gasbildung beteiligten Komponenten können die filmförmigen Zubereitungen zusätzlich einen oder mehrere Säureregulatoren enthalten. Hierfür kommen insbesondere Salze der Essigsäure, Natriumdihydrogen- oder Dinatriumhydrogenphosphat, Natriumtartrat und Natriumascorbat in Betracht.

Die Aktivierung der Gasproduktion erfolgt bevorzugt unter Einwirkung von Wasser, Feuchtigkeit oder eines wässrigen Mediums. Die filmförmigen Zubereitungen können darüber hinaus auch derartig aufgebaut sein, daß die Gasbildung durch veränderte thermische Bedingungen aktiviert wird, beispielsweise wenn eine filmförmige Zubereitung oral aufgenomnmen wird und dabei unter dem Einfluß der Körperwärme erwärmt wird (z. B. bei Erwärmung über 25 bis 35 °C). Eine Gaserzeugung durch thermische Aktivierung erzielt man mit Hilfe von Backtriebmitteln, zu denen Ammoniumcarbonat, Ammoniumhydrogencarbonat, Hirschhornsalz (eine Mischung aus Ammoniumcarbonat, Ammoniumhydrogencarbonat und Ammoniumcarbamat), sowie Hydrogencarbonat (Natriumhydrogencarbonat, Kaliumhydrogencarbonat) in Mischung mit sauren Phosphaten, sauren Pyrophosphaten, Citronensäure oder Weinsäure zählen.

Eine andere Möglichkeit, von der vorteilhaft Gebrauch gemacht werden kann, besteht darin, daß die Aktivierung der Gasbildung durch eine pH-Änderung ausgelöst wird, beispielsweise nach oraler Verabreichung einer filmförmigen Zubereitung. Durch den Zutritt von Feuchtigkeit bei Kontakt mit Speichel wird die Reaktion zwischen den gasbildenden Komponenten (Carbonat und/oder Hydrogencarbonat einerseits, Säurekomponente andererseits) ermöglicht. Die CO₂-freisetzende Reaktion beruht dabei auf einem Ansäuern (pH-Aktivierung) der Carbonat- bzw. Hydrogencarbonat-Komponente.

Unter "wässrigen Medien" werden im Zusammenhang mit der vorliegenden Erfindung insbesondere Wasser, wässrige Lösungen, Suspensionen, Dispersionen, wässrige Lösungsmittelgemische sowie physiologische Flüssigkeiten bzw. Körperflüssigkeiten (z. B. Körpersekrete, Speichel, Mucus) verstanden.

Aufgrund ihrer Befähigung zur Gasbildung zeichnen sich die erfindungsgemäßen filmförmigen Zubereitungen durch verbesserte Zerfallseigenschaften aus. Vorzugsweise sind diese Zubereitungen als schnell zerfallende Filme ausgestaltet, d. h. sie weisen Zerfallszeiten auf, die im Bereich von 1 s bis 5 min, vorzugsweise im Bereich von 1 s bis 1 min, besonders bevorzugt im Bereich von 1 s bis 30 s liegen.

Die filmförmigen Zubereitungen können eine Dicke im Bereich von 5 µm bis 3 mm, bevorzugt zwischen 10 µm und 1 mm, und besonders bevorzugt zwischen 20 µm und 500 µm aufweisen.

Im allgemeinen sind die erfindungsgemäßen filmförmigen Zubereitungen einschichtig aufgebaut. Allerdings ist gemäß einer bevorzugten Ausführungsform vorgesehen, daß die Zubereitungen aus mindestens zwei miteinander verbundenen Schichten aufgebaut sein können.

In einer besonderen Ausführungsform ist vorgesehen, daß die filmförmigen Zubereitungen in Wasser oder in wässrigen Medien quellfähig sind. Dies wird durch einen Gehalt an einer oder mehreren quellfähigen Substanzen erreicht, beispielsweise aus der Gruppe, die hydrophile Polyacrylate, hydrophile Polymethacrylate, anionische oder kationischeHydrogele, Agar, Carboxymethylcellulose, Methylcellulose, Tragant, Gelatine und quellfähige Ionenaustauscherharze umfaßt.

Die filmförmigen Zubereitungen eignen sich vorteilhaft als Darreichungsformen zur Verabreichung von pharmazeutischen Wirkstoffen. Deshalb ist in einer bevorzugten Ausführungsform vorgesehen, daß eine solche Zubereitung einen pharmazeutischen Wirkstoff, oder eine Kombination von zwei oder mehreren pharmazeutischen Wirkstoffen enthält. Der/die Wirkstoff(e) können in gelöster, dispergierter, suspendierter oder emulgierter Form vorliegen.

Optional können weitere freisetzbare Stoffe enthalten sein, z. B. Aromastoffe oder Süßstoffe.

Als Wirkstoffe kommen ohne Einschränkung solche Verbindungen in Betracht, die bei Mensch oder Tier therapeutisch wirksam sind. Diese können aus folgenden Gruppen stammen: Mittel zur Infektionsbehandlung, Virostatika, Analgetika wie Fentanyl, Sufentanil, Buprenorphin, Anesthetika, Anorectika, Wirkstoffe zur Behandlung von Arthritis und Asthma wie Terbutaline, Anticonvulsiva, Antidepressiva, Antidiabetika, Antihistaminika, Antidiarrhoika, Mittel gegen Migräne, Juckreiz, Übelkeit und Brechreiz, Reise bzw. Seekrankheit, wie Scopolamin und Ondansetron, Antineoplastika, Anti-Parkinson-Mittel, Antipsychotika, Antpyretika, Antispasmodika, Anticholinergika, Mittel gegen Ulkus wie Ranitidin, Sympathomimetika, Kalziumkanalblocker wie Nifedipin, Betablocker, Beta-Agonisten wie Dobutamin und Ritodrin, Antiarrythmika, Antihypertonika wie Atenolol, ACE-Hemmer wie Enalapril, Benzodiazepin-Agonisten wie Flumazenil, coronare, periphere und zerebrale Vasodilatoren, Stimulation für das Zentralnervensystem, Hormone, Hypnotika, Immunosuppressiva, muskelrelaxierende Mittel, Prostaglandine, Proteine, Peptide, Psychostimulanzien, Sedativa, Tranquilizer.
Geeignete Wirkstoffe finden sich ferner in den Wirkstoffgruppen der Parasympatholytika (z. B. Scopolamin, Atropin, Berlactyzin), der Parasympathomimetika, der Cholinergika (z. B. Physostigmin, Nicotin), der Neuroleptika (z. B. Chlorpromazin, Haloperidol), der Monoaminoxidasehemmer (z. B. Tranylcypromin, Selegilin), der Sympathomimetika (z. B. Ephedrin, D-Norpseudoephedrin, Salbutamol, Fenfluramin), der Sympatholytika und Antisympathotonika (z. B. Propranolol, Timolol, Bupranolol, Clonidin, Dihydroergotamin, Naphazolin), der Anxiolytika (z. B. Diazepam, Triazolam), der Lokalanästhetika (z. B. Lidocain), der zentralen Analgetika (z. B. Fentanyl, Sufentanil), der Antirheumatika (z. B. Indomethacin, Piroxicam, Lornoxicam), der Koronartherapeutika (z. B. Glyceroltrinitrat, Isosorbiddinitrat), der Östrogene, Gestagene und Androgene, der Antihistaminika (z. B. Diphenhydramin, Clemastin, Terfenadin), der Prostaglandinderivate, der Vitamine (z. B. Vitamin E, Cholecalciferol), der Cytostatika und der herzwirksamen Glykoside wie beispielsweise Digitoxin und Digoxin.

Die erfindungsgemäßen filmförmigen Zubereitungen können auch dazu verwendet werden, einen oder mehrere Aromastoffe, wie z. B. Menthol oder Lemon-Aroma, in der Mundhöhle freizusetzen. Der bzw. die Aromastoff(e) kann/können aber auch in Kombination mit einem oder mehreren pharmazeutischen Wirkstoffen in der Zubereitung vorliegen.

Der Wirkstoffgehalt bzw. der Gehalt an Aromastoff(en) beträgt vorzugsweise 0,1 bis 50 Gew.-%, besonders bevorzugt 1 bis 25 Gew.-%, jeweils bezogen auf die Trockenmasse einer filmförmigen Zubereitung.

Die Erfindung umfaßt ferner Verfahren, welche die Herstellung von filmförmigen, in wässrigen Medien zerfallsfähigen Zubereitungen ermöglichen, die gasbildende Komponenten enthalten und unter Einwirkung von Feuchtigkeit oder in Gegenwart eines wässrigen Mediums ein Gas erzeugen. Insbesondere eignen sich diese Verfahren zur Herstellung von filmförmigen Zubereitungen, wie sie in den Ansprüchen 7 und 8 und den davon abhängigen Ansprüchen beschrieben sind.

Die erfindungsgemäßen Herstellungsverfahren beruhen auf dem Grundprinzip, daß zunächst eine Beschichtungsmasse hergestellt wird, die Matrixpolymer(e), gasbildende(n) Stoff(e), Wirkstoff(e) und/oder Aromastoff(e) und gegebenenfalls weitere Hilfsstoffe enthält, und daß diese Beschichtungsmasse nachfolgend auf eine Unterlage beschichtet und danach getrocknet wird.

Bei der Herstellung der erfindungsgemäßen Zubereitungen ist der Umstand zu berücksichtigen, dass bei der Herstellung von wasserlöslichen (oder in Wasser zerfallsfähigen) Filmen in der Regel Wasser oder ein wässriges Medium als Lösungsmittel verwendet wird. Da die gasbildenden Komponenten durch Wasser oder Feuchtigkeit aktiviert werden, würde die gewünschte gasbildende Reaktion schon vorzeitig während der Herstellung einer Beschichtungsmasse erfolgen, welche die wasserlöslichen Matrixpolymere und die gasbildenden Komponenten enthält.

Erfindungsgemäß kann dieses Problem mit Hilfe der nachfolgend beschriebenen Maßnahmen gelöst werden:

Eine Möglichkeit zur Herstellung von filmförmigen Zubereitungen mit den beanspruchten Eigenschaften ergibt sich dadurch, daß zunächst aus zwei Beschichtungsmassen zwei Filme hergestellt werden, welche anschließend miteinander verbunden werden, wobei jede Beschichtungsmasse jeweils nur eine einzige der für die Gasbildung erforderlichen Komponenten enthält. Beispielsweise wird zunächst eine wässrige Polymerlösung mit Natriumhydrogencarbonat und eine weitere wässrige Polymerlösung mit Citronensäure hergestellt und durch Ausstreichen der Masse auf eine Prozessfolie als Unterlage (z. B. Polyesterfolie, Polyethylenfolie oder Metallfolie) und anschließende Trocknung jeweils ein wasserlöslicher Film hergestellt.
Da für die Aktivierung der Gaserzeugung beide Komponenten anwesend sein müssen, findet eine vorzeitige Gasbildung während der Herstellung der Beschichtungsmassen nicht statt, selbst wenn Wasser oder wässrige Medien als Löse-oder Suspendierungsmittel verwendet werden. Der gasbildende Prozess kann also nicht ablaufen, da die zur Gasbildung benötigten Komponente zunächst in getrennten Filmen vorliegen. Anschließend werden beide Filme - z.B. durch Kaschieren - vereinigt, so dass ein Film entsteht. Erst nach erfolgter Applikation (z. B. orale Verabreichung) nimmt die filmförmige Zubereitung Wasser auf, und die beiden gasbildenden Verbindungen kommen miteinander in Kontakt, wodurch die Gasbildung ausgelöst wird.

Dieses Herstellungsverfahren umfaßt die folgenden Schritte: Zunächst wird eine erste Beschichtungsmasse hergestellt, welche eine erste gasbildende Komponente sowie weitere Bestandteile der filmförmigen Zubereitung enthält. Dies kann durch Lösen, Suspendieren oder Dispergieren dieser Bestandteile in einem wässrigen Lösungs- oder Suspensionsmittel erfolgen. Auf entsprechende Weise wird eine zweite Beschichtungsmasse hergestellt, welche eine zweite gasbildende Komponente sowie weitere Bestandteile der filmförmigen Zubereitung enthält. Die erste und zweite Komponente sind notwendige Reaktionspartner einer gaserzeugenden Reaktion. Jede der beiden Beschichtungsmassen wird auf einer Unterlage ausgestrichen und getrocknet, wodurch ein erster und ein zweiter Film gebildet wird. Beide Filme werden durch Aufeinanderkaschieren vereinigt.

Ein weiteres erfindungsgemäßes Herstellungsverfahren macht von der Maßnahme Gebrauch, daß bei der Herstellung der Beschichtungsmasse(n) zumindest eine der gasbildenden Komponenten, oder ein Gemisch gasbildender Komponenten, in mikroverkapselter Form vorliegt. Durch eine derartige Verkapselung wird die gasbildende Reaktion während der Masseherstellung unterbunden. Erst bei der beispielsweise oralen Einnahme des Filmes kommt es - unter den im Mundraum gegebenen Umständen wie pH-Wert bzw. Körpertemperatur - zur Aktivierung der gasbildenden Reaktion. Geeignete Verfahren und Hilfsstoffe zur Mikroverkapselung von Partikeln sind dem Fachmann bekannt.

Das Verfahren sieht vor, daß eine Beschichtungsmasse hergestellt wird, welche die Bestandteile der Zubereitung einschließlich der gasbildenden Komponente enthält, wobei zumindest eine der gasbildenden Komponente in mikroverkapselter Form vorliegt. Die Herstellung der Beschichtungsmasse kann durch Lösen, Suspendieren oder Dispergieren der Bestandteile in einem Lösungs- oder Suspensionsmittel erfolgen. Da die Aktivierung der Gaserzeugung durch die Mikroverkapselung verhindert wird, können bei diesem Verfahren auch wässrige Medien als Lösungs- oder Suspensionsmittel eingesetzt werden. Anschließend wird die Beschichtungsmasse auf einer Unterlage ausgestrichen und getrocknet.

Die erfindungsgemäßen filmförmigen zerfallsfähigen Zubereitungen eignen sich vorteilhaft zur Verwendung als Darreichungsformen zur Verabreichung von pharmazeutischen Wirkstoffen zu therapeutischen Zwecken, insbesondere zur oralen, rektalen oder vaginalen Verabreichung.

Die Erfindung wird anhand der nachfolgenden Ausführungsbeispiele (nicht beansplucht) näher erläutert:

### Beispiel 1:

| Nr. | Bestandteil | Anteil Trockenmasse Gew.-% |
|---|---|---|
| 1 | Ethanol | |
| 2 | PVP | 33% |
| 3 | Zitronensäure | 20 % |
| 4 | NaBCO₃ | 35 % |
| 5 | Menthol | 7 % |
| 6 | Aspartam | 5 % |

### Beispiel 2:

| Nr. | Bestandteil | Anteil Trockenmasse Gew.-% |
|---|---|---|
| 1 | Ethanol | |
| 2 | HPC | 33% |
| 3 | Zitronensäure | 20 % |
| 4 | NaHCO₃ | 30 % |
| 5 | Lemon-Aroma | 12 % |
| 6 | Aspartam | 5 % |

### Herstellung der Massen für Beispiel 1 und 2:

Zunächst wird Nr. 1 vorgelegt und Nr. 2 unter Rühren hinzugegeben so dass eine 15%ige Polymerlösung entsteht. Anschließend werden Nr. 3, 5 und 6 hinzugegeben und gerührt bis die Masse homogen ist, danach wird Nr. 4 hinzugegeben und gerührt bis die Masse homogen ist. Die Masse wird als dünner Film auf eine Prozessfolie ausgestrichen und 15 Min. bei 60-80°C getrocknet. Der trockene Film wird dann zu Wafern vereinzelt.

## Patentansprüche

1. Verfahren zur Herstellung einer filmförmigen, in wässrigen Medien zerfallsfähigen Zubereitung zur Verabreichung von Stoffen an den menschlichen oder tierischen Körper, mit einem Gehalt an mindestens einem wasserlöslichen Polymer, wobei die Zubereitung eine oder mehrere Komponenten enthält, welche bei Einwirkung von Feuchtigkeit oder in Gegenwart eines wässrigen Mediums oder bei Temperaturänderung ein Gas erzeugen, durch Beschichtung einer Beschichtungsmasse oder zweier Beschichtungsmassen auf eine Unterlage, umfassend die Schritte
- Herstellen einer ersten Beschichtungsmasse, welche eine erste gasbildende Komponente sowie weitere Bestandteile der filmförmigen Zubereitung enthält, durch Lösen oder Suspendieren dieser Bestandteile in einem wässrigen Lösungs- oder Suspensionsmittel;
- Herstellen einer zweiten Beschichtungsmasse, welche eine zweite gasbildende Komponente sowie weitere Bestandteile der filmförmigen Zubereitung enthält, durch Lösen oder Suspendieren dieser Bestandteile in einem wässrigen Lösungs- oder Suspensionsmittel, wobei die erste und zweite gasbildende Komponente Reaktionspartner einer gaserzeugenden Reaktion sind;
- Ausstreichen der ersten Beschichtungsmasse auf einer Unterlage und Trocknen, wodurch ein erster Film gebildet wird;
- Ausstreichen der zweiten Beschichtungsmasse auf einer Unterlage und Trocknen, wodurch ein zweiter Film gebildet wird;
- Aufeinanderkaschieren der beiden Filme;
oder umfassend die Schritte:
- Herstellen einer Beschichtungsmasse, welche die Bestandteile der Zubereitung einschließlich des/der gasbildenden Komponente(n) enthält, durch Lösen oder Suspendieren der Bestandteile in einem Lösungs- oder Suspensionsmittel, wobei zumindest eine der gasbildenden Komponenten in mikroverkapselter Form vorliegt;
- Ausstreichen dieser Beschichtungsmasse auf einer Unterlage und Trocknen.

2. Verfahren nach Anspruch 1, **dadurch gekennzeichnet, daß** die gasbildends(n) Komponents(n) aus der Gruppe ausgewählt ist/sind, die Carbonate, insbesondere Natrlumcarbonat, Ammoniumcarbonat, Magnesiumcarbonat, Kallumcarbonat, und Hydrogencarbonate, lasbasonders Natriumhydrogencarbonat, und Säuren, insbesondere Citronensäure, Äpfelsäure, Esslgsäure, Milchsäure, Fumarsäure, Gluconsäure, Weinsäure, sowie Säureregulatoren, insbesonders Salze der Essigsäure, Natriumdlhydrogen-oder Dinstriumhydrogenphosphat, Natriurntartrat, Natriumascorbat, umfaßt.

3. Verfahren nach Anspruch 2, **dadurch gekennzeichnet, daß** als gasbildende Komponenten eine Komblnation aus mindestens einer Komponente (a) und mindestens einer Komponente (b) verwendet wird, wobei die Komponente(n)
(a) aus der Gruppe der Carbonsäuren, vorzugsweise aus der Citronensäure, Äpfeisäure, Essigsäure, Milchsäure, Fumarsäure, Gluconsäure und Weinsäure umfassenden Gruppe, und die Komponente(n).
(b) aus der Natriumhydrogencarbonat, Natriumcarbonat, Kallumcarbonat und Kaliumhydrogencarbonat umfassenden Gruppe ausgewählt ist/sind.

4. Verfahren nach einem der vorangehenden Ansprüche, **dadurch gekennzelchnet, daß** die Herstellung unter Zusatz eines pharmazeutischen Wirkstoffes oder einer Kombination von zwei oder mehreren pharmazeutischen Wirkstoffen erfolgt.

5. Verfahren nach einem der vorangehenden Ansprüche, **dadurch gekennzeichnet, daß** die Herstellung unter Zusatz eines Aromastoffes, vorzugsweise Menthol, erfolgt.

6. Fllmförmige, In wässrigen Medien zerfallsfählge Zubereitung, zur Verabreichung von Stoffen an den menschlichen oder tierischen Körper, mit einem Gehalt an mindestens einem wasserlöslichen Polymer, wobei die Zubereitung eine oder mehrere Komponennten enthält, welche bei Einwirkung von Feuchtigkeit oder In Gegenwart eines wässrigen Mediums oder bei Temperaturänderung ein Gas erzeugen, hergestellt nach einem der vorangehenden Ansprüche.

7. Filmförmige, in wässrigen Medien zerfallsfählge Zubereitung zur Verabreichung von Stoffen an den menschlichen oder tierischen Körper, mit einem Gehalt an mindestens einem wasserlösliche Polymer, wobei die Zubereitung eine oder mehrere Komponenten enthält, welche bei Einwirkung von Feuchtigkeit oder in Gegenwart eines wässrigen Mediums oder bei Temperaturänderung ein Gas erzeugen, **daduch gekennzeichnet, daß** zumindest eine der gasbildenden Komponenten in mikroverkapselter Form Jn der Zubereitung vorliegt.

8. Filmförmige, in wässrigen Medien zerfallsfähige Zubereitung zur Verabreichung von Stoffen an den menschlichen oder tierischen Körper, mit einem Gehalt an mindestens einem wasserlöslichen Polymer, wobei die Zubereitung eine oder mehrere Komponenten enthält, welche bei Einwirkung von Feuchtigkeit oder in Gegenwart eines wässrigen Mediums oder bei Temperaturänderung ein Gas erzeugen, **dadurch gekennzeichnet, daß** sie zwei miteinander verbundene Filmschichten aufweist, wobei die erste Filmschicht eine erste gasbildende Komponente sowie weitere Komponenten der filmförmigen Zubereitung enthält, und die zweite Filmschicht eine zweite gasbildende Komponente sowie weitere Komponenten der filmförmigen Zubereitung enthält, und wobei die erste und zweite gasbildende Komponente Reaktionspartner einer gaserzeugenden Reaktion sind.

9. Zubereitung nach Anspruch 7 oder 8, **dadurch gekennzeichnet, daß** die gasbildende(n) Komponente(n) aus der Gruppe ausgewählt ist/sind, die Carbonate, insbssondere Natriumcarbonat, Ammoniumcarbonat, Magnesiumcarbonat, Kaliumcarbonat, und Hydrogencarbonate, insbesondere Natriumhydrogencarbonat, und Säuren, insbesondere Citronensäure, Äpfelsäure, Essigsäure, Milchsäure, Fumarsäure, Gluconsäure, Welnsäure, sowie Säureregulatoren, insbesondere Salze der Essigsäure, Natriumdihydrogel- oder Dinatriumhydrogenphosphat, Natriumtarirat, Natriumascorbat, umfaßt.

10. Zubereitung nach Anspruch 9, **dadurch gekennzeichnet, daß** als gasbildende Komponenten eine Kombination aus mindestens einer Komponente (s) und mindestens einer Komponente (b) verwendet wird, wobei die Komponente(n)
(a) aus der Gruppe der Carbonsäuren, vorzugsweise aus der Citronensäure, Äpfelsäure, Essigsäure, Milchsäure, Fumarsäure, Gluconsäure und Weinsäure umfassenden Gruppe, und die Komponente(n)
(b) aus der Natrlumhydrogencarbonat, Natriumcarbonat, Kaliumcarbonat und Kaliumhydrogencarbonat umfassenden Gruppe ausgewählt ist/sind.

11. Zubereitung nach einem der Ansprüche 6 bis 10, **dadurch gekennzeichnet, daß** die Zubereitung befähigt ist, CO₂ oder N₂ zu erzeugen, vorzugsweise unter Einwirkung von Wasser oder eines wässrigen Mediums oder Feuchtigkeit.

12. Zubereitung nach einem der Ansprüche 6 bis 11, **dadurch gekennzeichnet, daß** die Zubereitung in Gegenwart von Wasser ein saures Milieu bildet.

13. Zubereitung nach einem der Ansprüche 6 bis 12, **dadurch gekennzeichnet, daß** die Zubereitung in Gegenwart von Wasser oder eines wässrigen Medlums innerhalb von 1 s bis 5 min, vorzugsweise innerhalb von 1 s bis 1 min, besonders bevorzugt innerhalb von 1 s bis 30 s, zerfällt.

14. Zubereitung nach einem der Ansprüche 6 bis 12, **dadurch gekennzeichnet, daß** die Zubereitung In wässrigen Medien queilfählg Ist.

15. Zubereitung nach einem der Ansprüche 6 bis 14, **dadurch gekennzelchnet, daß** die Zubereitung einen pharmazeutischen Wirkstoff oder eine Kombination von zwei oder mehreren pharmazeutischen Wirkstoffen enthält.

16. Zubereitung nach einem der Ansprüche 6 bis 15, **dadurch gekennzeichnet, daß** die Zubereltung einen Aromastoff, vorzugsweise Menthol, enthält.

17. Zubereitung nach einem der Ansprüche 6 bis 16, **dadurch gekennzeichnet, daß** die Zubereitung aus mindestens zwei Schichten aufgebaut ist.

18. Zubereitung nach einem der Ansprüche 6 bis 17, **dadurch gekennzeichnet, daß** die Zubereitung eine Dicke zwischen 5 µm und 3 mm, bevorzugt zwischen 10 µm und 1 mm, besonders bevorzugt zwischen 20 µm und 500 µm, aufweist.

19. Zubereitung nach einem der Ansprüche 6 bis 18, **dadurch gekennzeichnet, daß** sie als orale, rektale oder vaginale Darreichungsform zur Verabreichung von pharmazeutischen Wirkstoffen formullert ist.

## Claims

1. Process for the production of a film-shaped preparation for administration of substances to the human or animal body, said preparation being disintegratable in aqueous media and containing at least one water-soluble polymer and one or more components which produce a gas upon action of moisture or in the presence of an aqueous medium or in the case of a change in temperature, by means of coating a coating compound or two coating compounds on a support, comprising the steps of
- preparing a first coating compound which contains a first gas-forming component as well as further components of the film-forming preparation by dissolving or suspending said components in an aqueous solvent or suspending agent;
- preparing a second coating compound which contains a second gas-forming component as well as further components of the film-shaped preparation by dissolving or suspending said components in an aqueous solvent or suspending agent, said first and said second gas-forming component being reaction partners of a gas-forming reaction;
- spreading the first coating compound on a support and drying, thus forming a first film;
- spreading the second coating compound on a support and drying, thus forming a second film;
- laminating the two films onto each other;
or comprising the steps of:
- preparing a coating compound which contains the components of the preparation including the gas-forming component(s) by dissolving or suspending the said components in a solvent or a suspending agent, with at least one of the gas-forming components being present in microencapsulated form;
- spreading this coating compound on a support and drying.

2. Process according to claim 1, **characterized in that** the gas-forming component(s) is/are selected from the group comprising carbonates, especially sodium carbonate, ammonium carbonate, magnesium carbonate, potassium carbonate, and hydrogen carbonate, especially sodium hydrogen carbonate, and acids, especially citric acid, malic acid, acetic acid, lactic acid, fumaric acid, gluconic acid, tartaric acid, as well as acid regulators, especially salts of acetic acid, sodium dihydrogen phosphate or disodium hydrogen phosphate, sodium tartrate, sodium ascorbate.

3. Process according to claim 2, **characterized in that** as gas-forming components a combination of at least one component (a) and at least one component (b) is used, said component(s)
(a) being selected from the group of the carboxylic acids, preferably from the group comprising citric acid, malic acid, acetic acid, lactic acid, fumaric acid, gluconic acid and tartaric acid, and said components
(b) being selected from the group comprising sodium hydrogen carbonate, sodium carbonate, potassium carbonate and potassium hydrogen carbonate.

4. Process according to any one of the preceding claims, **characterized in that** the production is accomplished under addition of a pharmaceutical active substance or a combination of two or more pharmaceutical active substances.

5. Process according to any one of the preceding claims, **characterized in that** the production is accomplished under addition of a flavouring agent, preferably menthol.

6. Film-shaped preparation disintegratable in aqueous media, for administration of substances to the human or animal body, containing at least one water-soluble polymer, said preparation containing one or more components which produce a gas upon action of moisture or in the presence of an aqueous medium or in the case of a change in temperature, produced in accordance with any one of the preceding claims.

7. Film-shaped preparation disintegratable in aqueous media, for administration of substances to the human or animal body, containing at least one water-soluble polymer, said preparation containing one or more components which produce a gas upon action of moisture or in the presence of an aqueous medium or in the case of a change in temperature, **characterized in that** at least one of the gas-forming components is present in microencapsulated form.

8. Film-shaped preparation disintegratable in aqueous media, for administration of substances to the human or animal body, containing at least one water-soluble polymer, said preparation containing one or more components which produce a gas upon action of moisture or in the presence of an aqueous medium or in the case of a change in temperature, **characterized in that** it has two film layers which are connected with each other, the first film layer containing a first gas-forming component as well as further components of the film-shaped preparation, and the second film layer containing a second gas-forming component as well as further components of the film-shaped preparation, and said first and second gas-forming components being reaction partners of a gas-forming reaction.

9. Preparation according to claim 7 or 8, **characterized in that** the gas-forming component(s) is/are selected from the group comprising carbonates, especially sodium carbonate, ammonium carbonate, magnesium carbonate, potassium carbonate, and hydrogen carbonate, especially sodium hydrogen carbonate, and acids, especially citric acid, malic acid, acetic acid, lactic acid, fumaric acid, gluconic acid, tartaric acid, as well as acid regulators, especially salts of acetic acid, sodium dihydrogen phosphate or disodium hydrogen phosphate, sodium tartrate, sodium ascorbate.

10. Preparation according to claim 9, **characterized in that** as gas-forming components a combination of at least one component (a) and at least one component (b) is used, said component(s)
(a) being selected from the group of the carboxylic acids, preferably from the group comprising citric acid, malic acid, acetic acid, lactic acid, fumaric acid, gluconic acid and tartaric acid, and said components
(b) being selected from the group comprising sodium hydrogen carbonate, sodium carbonate, potassium carbonate and potassium hydrogen carbonate.

11. Preparation according to any one of claims 6 to 10, **characterized in that** said preparation is capable of producing CO₂ or N₂, preferably under action of water or an aqueous medium or moisture.

12. Preparation according to any one of claims 6 to 11, **characterized in that** it produces an acid environment in the presence of water.

13. Preparation according to any one of claims 6 to 12, **characterized in that** said preparation disintegrates in the presence of water or an aqueous medium within 1 s to 5 min, preferably within 1 s to 1 min, especially preferably within 1 s to 30 s.

14. Preparation according to any one of claims 6 to 12, **characterized in that** said preparation is swellable in aqueous media.

15. Preparation according to any one of claims 6 to 14, **characterized in that** said preparation contains a pharmaceutical active substance or a combination of two or more pharmaceutical active substances.

16. Preparation according to any one of claims 6 to 15, **characterized in that** said preparation contains a flavouring agent, preferably menthol.

17. Preparation according to any one claims 6 to 16, **characterized in that** said preparation comprises at least two layers.

18. Preparation according to any one of claims 6 to 17, **characterized in that** said preparation has a thickness between 5 µm and 3 mm, preferably between 10 µm and 1 mm, especially preferably between 20 µm and 500 µm.

19. Preparation according to any one of claims 6 to 18, **characterized in that** it is formulated as an oral, rectal or vaginal administration form for administration of pharmaceutical active agents.

## Revendications

1. Procédé pour la fabrication d'une préparation filmogène, apte à se décomposer dans des milieux aqueux pour l'administration de substances sur le corps humain ou animal, possédant une teneur en au moins un polymère soluble dans l'eau, la préparation contenant un ou plusieurs composants qui génèrent un gaz sous l'effet de l'humidité ou en présence d'un milieu aqueux ou encore dans le cas d'une modification de la température, par enduction d'une matière d'enduction ou de deux matières d'enduction sur un substrat, comprenant les étapes
- de fabrication d'une première matière d'enduction qui contient un premier composant formateur de gaz ainsi que d'autres constituants de la préparation filmogène, par dissolution ou par mise en suspension de ces constituants dans un solvant aqueux ou dans un agent aqueux de mise en suspension ;
- de fabrication d'une deuxième matière d'enduction qui contient un deuxième composant formateur de gaz ainsi que d'autres constituants de la préparation filmogène, par dissolution ou par mise en suspension de ces constituants dans un solvant aqueux ou dans un agent aqueux de mise en suspension, le premier et le deuxième composants formateurs de gaz représentant des partenaires réactionnels d'une réaction générant un gaz ;
- d'étalement de la première matière d'enduction sur un substrat et de séchage, pour ainsi obtenir un premier film ;
- d'étalement de la deuxième matière d'enduction sur un substrat et de séchage, pour ainsi obtenir un deuxième film ;
- de contrecollage des deux films l'un à l'autre ;
ou comprenant les étapes :
- de fabrication d'une matière d'enduction qui contient les constituants de la préparation y compris du/des composant(s) formateur(s) de gaz, par dissolution ou mise en suspension des constituants dans un agent de dissolution ou de mise en suspension, au moins un des composants formateurs de gaz étant présent sous une forme microencapsulée ;
- d'étalement de cette matière d'enduction sur un substrat et de séchage.

2. Procédé selon la revendication 1, **caractérisé en ce que** le/les composant(s) formateur(s) de gaz est/sont choisi(s) parmi le groupe qui comprend des carbonates, en particulier le carbonate de sodium, le carbonate d'ammonium, le carbonate de magnésium, le carbonate de potassium et des hydrogénocarbonates, en particulier l'hydrogénocarbonate de sodium, et des acides, en particulier l'acide citrique, l'acide malique, l'acide acétique, l'acide lactique, l'acide fumarique, l'acide gluconique, l'acide tartrique, ainsi que des régulateurs de l'acidité, en particulier des sels de l'acide acétique, le dihydrogénophosphate de sodium ou l'hydrogénophosphate disodique, le tartrate de sodium, l'ascorbate de sodium.

3. Procédé selon la revendication 2, **caractérisé en ce qu'**on utilise, à titre de composant formateur de gaz, une combinaison d'au moins un composant (a) et d'au moins un composant (b), le/les composants (a) étant choisi(s) parmi le groupe des acides carboxyliques, de préférence parmi le groupe comprenant l'acide citrique, l'acide malique, l'acide acétique, l'acide lactique, l'acide fumarique, l'acide gluconique et l'acide tartrique, et le/les composants (b) étant choisi(s) parmi le groupe comprenant l'hydrogénocarbonate de sodium, le carbonate de sodium, le carbonate de potassium et l'hydrogénocarbonate de potassium.

4. Procédé selon l'une quelconque des revendications précédentes, **caractérisé en ce que** la fabrication a lieu par addition d'une substance active pharmaceutique ou d'une combinaison de deux substances actives pharmaceutiques ou plus.

5. Procédé selon l'une quelconque des revendications précédentes, **caractérisé en ce que** la fabrication a lieu par addition d'une substance aromatique, de préférence de menthol.

6. Préparation filmogène, apte à se décomposer dans des milieux aqueux pour l'administration de substances sur le corps humain ou animal, possédant une teneur d'au moins un polymère soluble dans l'eau, la préparation contenant un ou plusieurs composants qui génèrent un gaz sous l'effet de l'humidité ou en présence d'un milieu aqueux ou encore dans le cas d'une modification de la température, que l'on fabrique conformément à l'une quelconque des revendications précédentes.

7. Préparation filmogène, apte à se décomposer dans des milieux aqueux pour l'administration de substances sur le corps humain ou animal, possédant une teneur d'au moins un polymère soluble dans l'eau, la préparation contenant un ou plusieurs composants qui génèrent un gaz sous l'effet de l'humidité ou en présence d'un milieu aqueux ou encore dans le cas d'une modification de la température, **caractérisée en ce qu'**au moins un des composants formateurs de gaz est présent dans la préparation sous une forme microencapsulée.

8. Préparation filmogène, apte à se décomposer dans des milieux aqueux pour l'administration de substances sur le corps humain ou animal, possédant une teneur d'au moins un polymère soluble dans l'eau, la préparation contenant un ou plusieurs composants qui génèrent un gaz sous l'effet de l'humidité ou en présence d'un milieu aqueux ou encore dans le cas d'une modification de la température, **caractérisée en ce qu'**elle présente deux couches minces reliées l'une à l'autre, la première couche mince contenant un premier composant formateur de gaz ainsi que d'autres composants de la préparation filmogène, et la deuxième couche mince contenant un deuxième composant formateur de gaz ainsi que d'autres composants de la préparation filmogène, le premier et le deuxième composants formateurs de gaz représentant des partenaires réactionnels d'une réaction générant un gaz.

9. Préparation selon la revendication 7 ou 8, **caractérisée en ce que** le/les composant(s) formateur(s) de gaz est/sont choisi(s) parmi le groupe qui comprend des carbonates, en particulier le carbonate de sodium, le carbonate d'ammonium, le carbonate de magnésium, le carbonate de potassium et des hydrogénocarbonates, en particulier l'hydrogénocarbonate de sodium, et des acides, en particulier l'acide citrique, l'acide malique, l'acide acétique, l'acide lactique, l'acide fumarique, l'acide gluconique, l'acide tartrique, ainsi que des régulateurs de l'acidité, en particulier des sels de l'acide acétique, le dihydrogénophosphate de sodium ou l'hydrogénophosphate disodique, le tartrate de sodium, l'ascorbate de sodium.

10. Préparation selon la revendication 9, **caractérisée en ce qu'**on utilise, à titre de composant formateur de gaz, une combinaison d'au moins un composant (a) et d'au moins un composant (b), le/les composants (a) étant choisi(s) parmi le groupe des acides carboxyliques, de préférence parmi le groupe comprenant l'acide citrique, l'acide malique, l'acide acétique, l'acide lactique, l'acide fumarique, l'acide gluconique et l'acide tartrique, et le/les composants (b) étant choisi(s) parmi le groupe comprenant l'hydrogénocarbonate de sodium, le carbonate de sodium, le carbonate de potassium et l'hydrogénocarbonate de potassium.

11. Préparation selon l'une quelconque des revendications 6 à 10, **caractérisée en ce que** la préparation est à même de générer du CO₂ ou du N₂, de préférence sous l'influence de l'eau ou d'un milieu aqueux ou de l'humidité.

12. Préparation selon l'une quelconque des revendications 6 à 11, **caractérisée en ce que** la préparation forme un milieu acide en présence d'eau.

13. Préparation selon l'une quelconque des revendications 6 à 12, **caractérisée en ce que** la préparation se décompose en présence d'eau ou d'un milieu aqueux dans un laps de temps de 1 s à 5 min., de préférence dans un laps de temps de 1 s à 1 min., de manière particulièrement préférée dans un laps de temps de 1 s à 30 s.

14. Préparation selon l'une quelconque des revendications 8 à 12, **caractérisée en ce que** la préparation est à même de gonfler dans des milieux aqueux.

15. Préparation selon l'une quelconque des revendications 6 à 14, **caractérisée en ce que** la préparation contient une substance active pharmaceutique ou une combinaison de deux substances actives pharmaceutiques ou plus.

16. Préparation selon l'une quelconque des revendications 8 à 15, **caractérisée en ce que** la préparation contient une substance aromatique, de préférence du menthol.

17. Préparation selon l'une quelconque des revendications 6 à 16, **caractérisée en ce que** la préparation est constituée par au moins deux couches.

18. Préparation selon l'une quelconque des revendications 6 à 17, **caractérisée en ce que** la préparation présente une épaisseur entre 5 µm et 3 mm, de préférence entre 10 µm et 1 mm, de manière particulièrement préférée entre 20 µm et 500 µm.

19. Préparation selon l'une quelconque des revendications 6 à 18, **caractérisée en ce que** la préparation est formulée à titre de forme d'administration orale, rectale ou vaginale pour l'administration de substances actives pharmaceutiques.
